Europäisches Patentamt

European Patent Office

Office européen des brevets

(11) Publication number: **0 074 407**
**B1**

# EUROPEAN PATENT SPECIFICATION

(45) Date of publication of patent specification: **16.06.87**

(51) Int. Cl.⁴: **A 61 B 5/22**

(21) Application number: **81900712.1**

(22) Date of filing: **18.03.81**

(86) International application number:
**PCT/JP81/00059**

(87) International publication number:
**WO 82/03166 30.09.82 Gazette 82/23**

(54) Apparatus for measuring the extent of muscle fatigue or pain.

(43) Date of publication of application:
**23.03.83 Bulletin 83/12**

(45) Publication of the grant of the patent:
**16.06.87 Bulletin 87/25**

(84) Designated Contracting States:
**DE FR GB**

(56) References cited:
**US-A-3 916 876**

**JOURNAL OF APPLIED MECHANICS, vol. 43,
no. 1, March 1976, NEW YORK (US) G.I.
ZAHALAK et al.: "Partially Activated Human
Skeletal Muscle: An Experimental
Investigation of Force, Velocity, and EMG.",
pages 81-86**

**JOURNAL OF PHYSIOLOGY, vol. 123, 1954,
(GB) B. BIGLAND et al.: "The relation between
force, velocity and integrated electrical activity
in human muscles.", pages 214-224
ENGINEERING IN MEDICINE, vol. 9, no. 3, July
1980 LONDON (GB) E.G. WALSH et al.:**

(73) Proprietor: **KONNO, Yoshio**
**12-18, Jinnan 1-chome Shibuya-ku
Tokyo 150 (JP)**

(72) Inventor: **KONNO, Yoshio**
**12-18, Jinnan 1-chome Shibuya-ku
Tokyo 150 (JP)**

(74) Representative: **Cockbain, Julian Roderick
Michaelson et al
Frank B. Dehn & Co. European Patent Attorneys
Imperial House 15-19, Kingsway
London WC2B 6UZ (GB)**

(56) References cited:

**"Measurements of muscle tone using printed
motors as torque generators", pages 167-171
BIOMEDIZINISCHE TECHNIK, vol. 22, no. 4,
April 1977 BERLIN (DE) A. FERRAIOLI: "Signal
to noise ratio of the filtered electromyographic
signal (EMG) in the isometric muscle
contraction", pages 86-92**

**IEEE TRANSACTIONS ON BIOMEDICAL
ENGINEERING, vol. BME-28, no. 4, April 1981
NEW YORK (US) F. TANZI et al.: "Spectral
Analysis of Surface Motor Unit Action
Potentials and Surface Interference
Electromyogram", pages 318-324**

## Description

The present invention relates to an apparatus for measuring the extent of muscle fatigue or pain using measurements of muscular strength and myoelectric discharge and in particular to such apparatus arranged to measure the extent of muscle fatigue or pain by determining a ratio of integrated detected electromyogrammatic and muscle strength signals.

In the conventional load electromyogram used for measurement of fatigue by an electromyogram, a load of constant value was used. For example, the experiment on arm bending utilizing an ergonometer was an experiment to investigate the fatigue resulting from the repeated raising of a weighted body to a definite position and not an experiment to investigate the muscular strength and the electromyogram in the process of reaching the maximum muscular strength. It was also impossible to know whether or not, at the time of measurement, the subject was already tired or felt pain and, if he was tired or felt pain, what its degree was.

The present inventor reported a method of recording, as a muscular strength electromyogram on a recorder, the discharge amount derived from a muscle being tested and the muscular strength of the muscle being tested. The method involved on the one hand passing the discharge amount through an input means and an electromyogram amplifier and on the other hand passing the muscular strength through a transducer to convert it into an electric signal and supplying that signal to an amplifier, the muscular strength and electromyogrammatic signals from these amplifiers being supplied to a recorder amplifier and then to a recorder (see "Industrial Medicine", Vol. 18, No. 4, 1976, pages 383 to 390). It has been found from the muscular strength electromyogram obtained by this method that the amplitude of the electromyogram increases as the muscular strength increases and the discharge amount of the muscle at the time of fatigue is higher than that when not tired. It has also been found that when the muscle is dead tired, the discharge amount increases considerably in spite of reduced muscular strength. However, this method has the problem that degrees of fatigue cannot be exactly compared numerically.

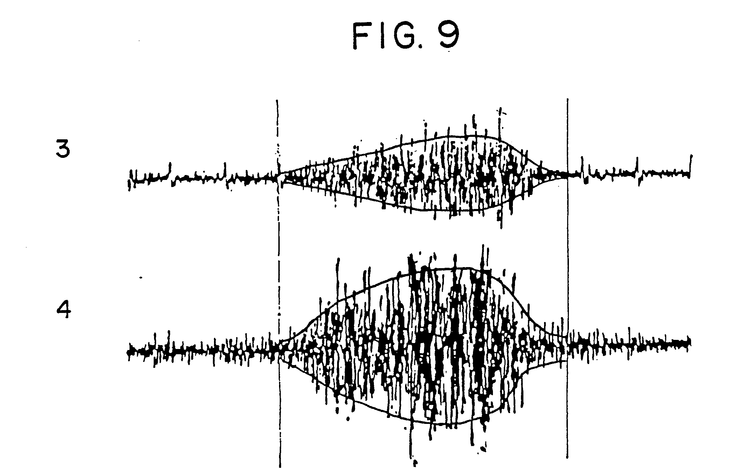

The present inventor also found it necessary to find the areas of muscular strength electromyograms and to obtain the ratio therebetween in order to compare exactly the degree of fatigue of muscles based on the muscular strength electromyograms. A method of measuring and comparing the areas of electromyograms was reported by him in "Industrial Medicine", Vol. 20, No. 2, 1978, pages 94 to 104. According to this method, the areas of electromyograms can be found by forming the envelopes thereof as shown in Fig. 9 hereto. However, this method has the disadvantages that not only is there a considerable error in drawing envelopes due to differences depending on the individuals drawing the envelopes but also the work involved is time-consuming and troublesome.

It has now been found that shortcomings of the prior art methods may be overcome by determining a ratio between the integrals of the electromyogrammatic and the muscular strength signals, this ratio being representative of the extent of fatigue or pain in the muscle being examined.

The present invention thus provides an apparatus for measuring the extent of muscle fatigue or pain using measurements of muscular strength and myoelectric discharge, characterized in that said apparatus comprises means for transforming the muscular strength of a muscle being examined into an electrical muscular strength signal, means for detecting a myoelectric discharge from said muscle to generate an electromyogrammatic signal, first integrator means for integrating said electromyogrammatic signal, second integrator means for integrating said muscular strength signal, and ratio determining means for determining the ratio between the integrated electromyogrammatic signal and the integrated muscular strength signal.

The muscular strength, however, is dependent on the constitution or physique of the subject and the constitution can be considered to be represented by the subject's body weight. Thus, since a person of a lighter weight has a lower muscular strength than a person of a heavier weight, the ratio between the integrated values of the muscular strength and electromyogrammatic signals is smaller for the lighter person for the same integrated values of the electromyogrammatic signal. This fact suggests that the numerical values, i.e. the measured ratios of integrated electromyogrammatic and muscular strength signals cannot be compared directly between subjects of different weights. However, in a preferred embodiment of the apparatus of the invention, the apparatus is provided with means for adjusting the ratio between the integrated electromyogrammatic signal and the integrated muscular strength signal by a weight-muscular strength coefficient to provide an adjusted ratio which can be used in the comparison between subjects of different weights. Conveniently, the means for adjusting the ratio will comprise means for adjusting the muscular strength signal before the integration thereof.

Operation of the apparatus of the invention involves determining the variation in the muscular strength (such as dorsal muscular strength, shoulder arm strength, grasping power, or the like) through a transducer as an electric signal and, at the same time, determining the electromyogrammatic waveform induced from the necessary part of the muscle being tested, the variation in the muscular strength and the electromyogrammatic wave form conveniently being processed for digital representation of the maximum muscular strength and the maximum amplitude of the electromyogrammatic signal and of the weight adjusted ratio between the integrated values of the electromyogrammatical and muscular strength signals.

The apparatus of the present invention employs dorsal muscular strength, shoulder arm strength, grasping power meter and applies electrodes to right and left neck regions, an upper part of the trapezius muscle, the deltoid muscle, an interscapular region or space, a lumbodorsal region, the petcoralis major muscle, the antebrachial muscle, etc. to induce and measure the myoelectric.

Since electric signals obtained from these parts cannot be displayed digitally as they are, they are conveniently subjected to waveform processing. Thus, the apparatus of the invention preferably comprises as said means for detecting a myoelectric discharge (i) a pair of electrodes adapted to be placed on the muscle to be examined to generate a raw electromyogrammatic signal and (ii) waveform processing means comprising filter means for filtering out components of the electromyogrammatic signal having a frequency outside a desired range, rectifier means and conversion means for converting the filtered and rectified electromyogrammatic signal into an envelope signal for subsequent integration by said first integrator means. Conveniently in the waveform processing means the electromyogrammatic signal components having frequencies of 10 Hz or less and 150 Hz or more, which do not appear on an electromyogrammatic meter, are removed by means of a high-pass filter and a low-pass filter and then the filtered signals are transformed by a full-wave rectifier circuit into waveforms so that they can be easily supplied to a digital processor. Then, envelope signals are formed from these waveforms and passed through a low-pass filter which passes 3 Hz or less to get rid of fine variations and finally supplied to a display.

Separately therefrom, the signal derived from the muscular strength through a transducer and an amplifier is supplied to a processing indicator through a delay circuit to be processed after being delayed by the time equal to the delay time resulting from the waveform processing.

While a person of less fatigue has a higher muscular strength and a lower discharge amount (i.e integrated electromyogrammatic signal), a person of more fatigue or pain has a lower muscular strength and a higher discharge amount. To know the degree of fatigue or pain it is necessary to find the ratio of the discharge amount to the muscular strength (AMS ratio). However, since the muscular strength depends on the constitution or physique, to make a comparison between subjects on the same basis it is necessary to effect a weight correction.

The increase in the muscular strength is not proportional to that in the weight, but its ratio (weight/muscular strength coefficient) is proportional to the weight raised to the power of 2/3. Thus, when the reference weight is assumed to be 50 Kg the weight correction value is

$$P = 50 \times (W/50)^{2/3}$$

where

P=weight correction value
W=weight.

The progressive tendency of this correction value is that it is 50 for a weight of 50 Kg, 56.5 for 60 Kg, 62.8 for 70 Kg, 68.4 for 80 Kg, 74.0 for 90 Kg, 79.4 for 100 Kg, and 43.1 for 40 Kg, 35.6 for 30 Kg, and 27.1 for 20 Kg.

When the weight correction is made with these values, a discharge amount/muscular strength ratio is multiplied by P/50. The correction factor is $\times 1$ for the weight of 50 Kg and $\times 79.4/50$ (=1.59) for 100 Kg, for example. The above mentioned reference weight is not necessarily 50 Kg. It can be replaced by any numerical value. Thus, the adjusted ratio of the integrated electromyogrammatic signal to the integrated muscle strength signal may be achieved by multiplying the muscle strength signal by $W_0/P$ where $P=W_0(W/W_0)^{2/3}$, W is the subject's weight and $W_0$ is a reference weight.

Since the AMS ratio obtained by this method has been affected by a weight correction, the fatigue and pain of subjects of different weights can be compared on the basis of the same criterion.

Thus, use of apparatus according to the invention permits the digital representation of muscular strength electromyograms so that subjects of different constitutions can be compared on the same basis by numerically expressing the fatigue or pain of muscles. The apparatus preferably functions to display on a panel display the muscular strength (Kg), the maximum electromyogrammatic discharge amount (mV) of the muscle being tested and the ratio between the integrals of the electromyogrammatic and muscular strength signals. This may be achieved by passing the muscular strength (such as dorsal muscular strength, shoulder arm strength and grasping power) through a transducer to provide an electrical muscular strength signal, using an amplifier to amplify the signal, and by using an electromyogram amplifier coupled with detection electrodes to derive an action potential from the part of the muscle being tested, and a waveform processor for processing the waveform of the electromyogrammatic signal.

A preferred embodiment of the apparatus of the present invention will now be described by way of example with reference to the accompanying drawings, in which:—

Fig. 1 is a block diagram of an apparatus according to the present invention;
Fig. 2 is a block diagram of a waveform processor for the apparatus of Fig. 1;
Fig. 3 is a block diagram of a digital processor for the apparatus of Fig. 1;
Fig. 4 is a diagram of a weight correction circuit for the apparatus of Fig. 1;
Fig. 5 shows diagrammatically the electromyogrammatic signals and the muscular strength signal determined for a subject with apparatus according to the present invention as well as the locations of electromyogrammatic signal detection on the subject's body;
Figs. 6 to 8 show diagrammatically digital displays of the apparatus of the invention for the subject of Fig. 5; and

Fig. 9 is a diagram showing the prior art method of finding the areas of electromyogrammatic waveforms (here waveforms 3 and 4 of Fig. 5) by means of envelopes.

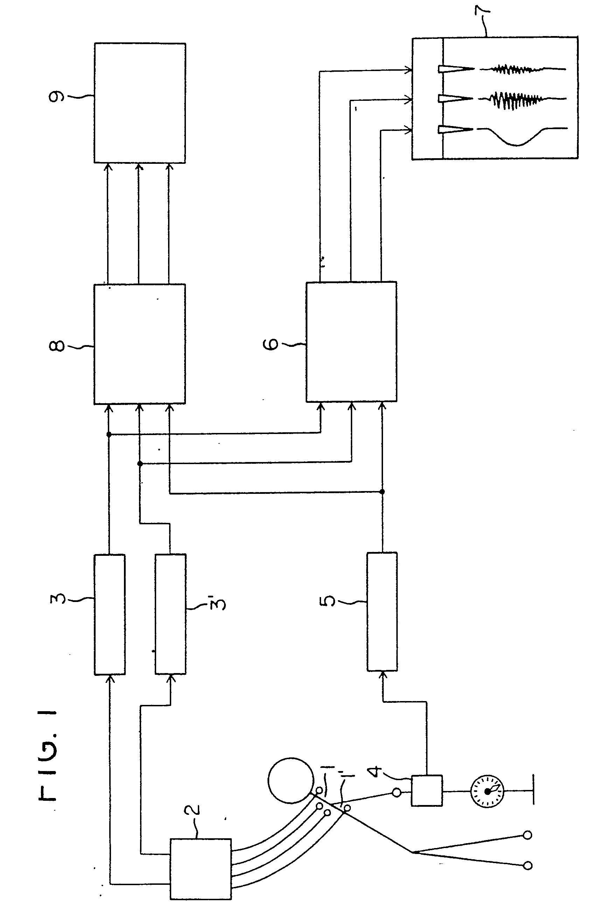

Referring to Fig. 1, there is shown a block diagram of a dorsal muscular strength meter for the measurement of muscular strength. An action potential is picked up from the muscle to be tested by surface electrodes 1 and 1' applied thereto and supplied through an input part 2 to the electromyogram amplifiers 3 and 3' for amplification. Apart therefrom the muscular strength of the muscle to be tested is transformed using a transducer 4 into an electric signal which is then supplied to an amplifier 5. Muscular strength and electromyogrammatic signals derived from the devices 3, 3' and 5 are recorded on a penrecorder 7 as a muscular strength diagram and electromyograms through a recorder amplifier 6. Separately therefrom, the electromyogrammatic signals and the muscular strength signal from the devices 3, 3' and 5 are supplied to a waveform processor 8 to facilitate the proper supply of the maximum values of the electromyogrammatic signals and the muscular strength signals to a digitally processing display 9 and, at the same time, to facilitate determination of the ratio between the integrated values of the electromyogrammatic signals and the muscular strength signals.

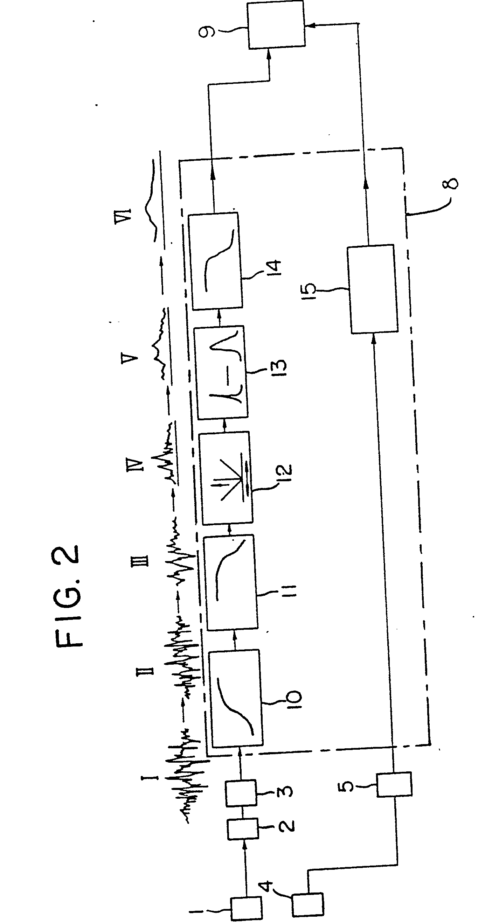

Fig. 2 is a block diagram illustrating the operation of this waveform processor. The signal I produced by the devices 3 and 3' is shaped into a signal of waveform II and III by removing therefrom by means of a high-pass filter (10) and a low-pass filter (11) respectively low signal components of 10 Hz or less due to the movement of the electrode and the myoelectric and high signal components of 150 Hz or more which are not recorded on a pen-recorder as an electromyogram. The electromyogrammatic signal shaped into waveforms II and III is transformed by a full-wave rectifier circuit 12 into a signal of waveform IV to facilitate its supply to a digitally processing display 9.

If the rectified electromyogrammatic signal of waveform IV is supplied directly to the processing display 9, merely an average value of the signal is recorded which is of a different value from that visually observed on a penrecorder. Therefore, the rectified electromyogrammatic signal of waveform IV is converted into an envelope signal of waveform V using a peak hold circuit 13 which has a discharge time longer than the charging time. The signal of waveform V is further passed through a low-pass filter 14 which passes signals of 3 Hz or less to get rid of fine variations and then is supplied to the processing display 9.

Separately therefrom, the muscular strength signal picked up through the transducer 4 and the amplifier 5 is delayed by a delay circuit 15 by a time equal to the time delay due to the wave processing and then is supplied to the processing display 9.

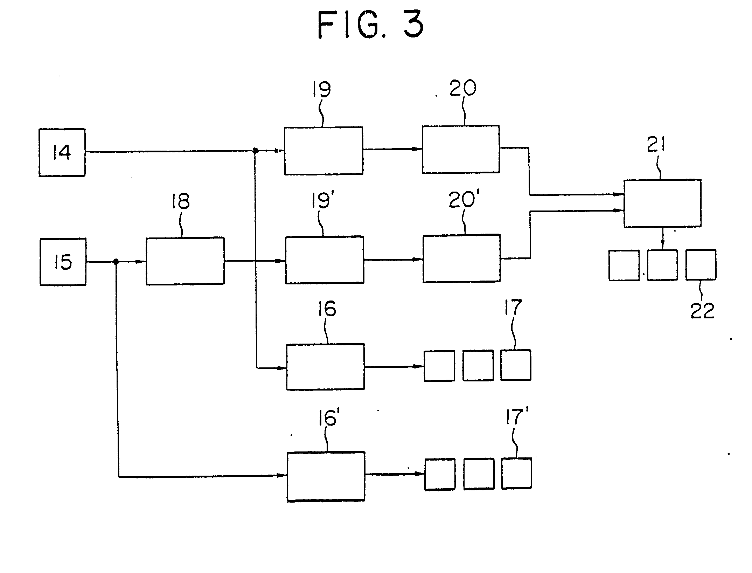

Fig. 3 is a block diagram of the digitally processing display 9. A part of the signal which was waveform-processed by the low-pass filter 14 in Fig. 2, after passing through a frequency modulator 19, is counted by a counter circuit 20 and the signal from the counter circuit 20 is supplied to a divider 21. The rest of the signal from the low-pass filter 14 passes through a maximum detection circuit 16 and enters and electromyogram maximum value indicator 17 where the maximum amplitude of the waveform-processed electromyogrammatic signal is indicated by a numerical value of three digits.

A part of the muscular strength signal from the delay circuit 15, after passing through a correction circuit 18 and a frequency modulator 19', enters a counter 20' where it is counted and the signal from counter 20' enters the divider 21. The rest of the signal from the delay circuit 15 passes through a maximum value detection circuit 16' and then enters a muscular strength maximum value indicator 17' where the maximum muscular strength is indicated by a numerical value of three digits.

From the signals supplied from the counter circuits 20 and 20', divider 21 processes the ratio between the integrated values provided by the low-pass filter 14 and by the delay circuit 15 and correction circuit 18. The processed ratio is indicated on an indicator 22 as a numerical value of three digits. Thus, in order to cause the start and the end of integration processing of the muscular strength and the electromyogram at the same time, an adjustment is made such that the integration starts when the muscular strength rises to a set level from the zero level and stops when the muscular strength returns to the same set level.

The numerical values indicated on the muscular strength maximum value indicator 17' and the electromyogram maximum value indicator 17 show under what functional conditions of the body the ratio between the integrated values indicated on the indicator 22 was obtained. For example, for the same ratio between the integrated values, the muscular strength and the electromyogram of a subject of inexhaustible vitality show high numerical values, whereas those of a subject of reduced vitality show low numerical values. Consequently, while the numerical value of the ratio between the integrated values alone cannot enable judgement of functional conditions of a subject, the indication of the maximum values of the muscular strength and the electromyogram enables the functional conditions of the body to be known.

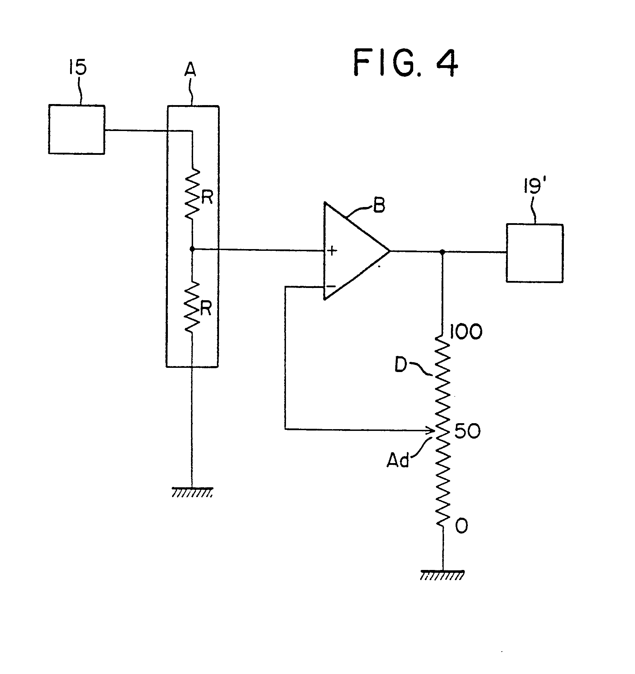

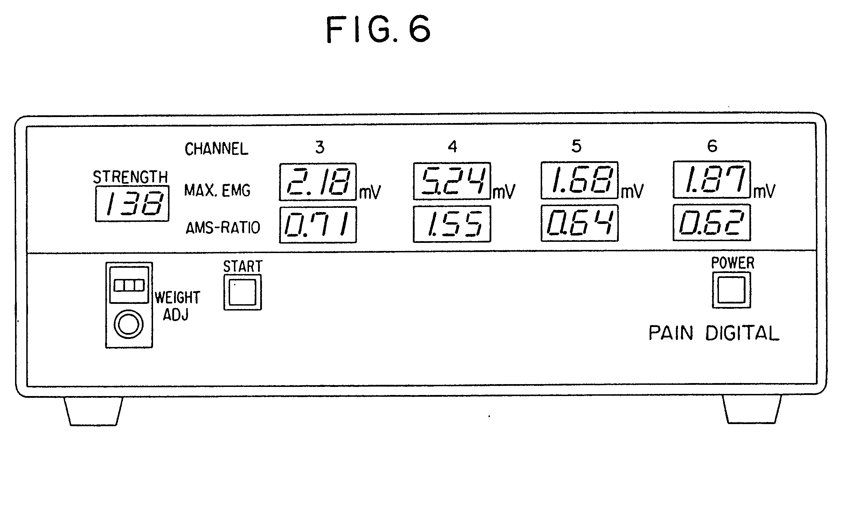

Fig. 4 shows a muscular strength correction circuit 18 for correcting the muscular strength with the body weight. A muscular strength signal derived from the delay circuit 15 is supplied to an attenuator A the output of which is supplied to an amplifier B. The adjusting point Ad of a divider D is set at a point higher or lower than 50 by the output of the amplifier B depending on the adjustment value P at the weight adjuster on the panel of Figs. 6 to 8. Thus, the signal supplied from the delay circuit 15 is amplified 50/P times and divided and indicated through the frequency modulator 19' and the counter circuit 20'. The correction value P can also be set automatically by inputting the weight of the subject.

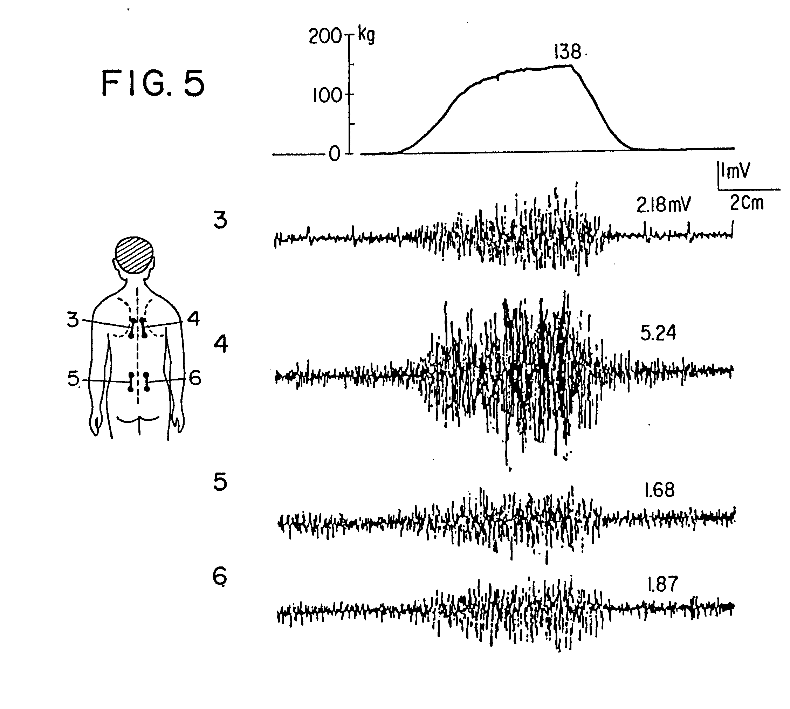

Examples of measurements of the extent of muscle fatigue or pain using apparatus according to the present invention will be described with reference to Figs. 5 to 8. The electrodes employed were dish shaped surface electrodes which were applied to the parts to be tested at an interval of 50 mm. The calibration voltage was 40 mm/200 Kg for the dorsal muscular strength an 10 mm/1 mV for the electromyogram, and the time constant was 0.01 sec.

Fig. 5 is a record of the result of measurement of the dorsal muscular strength electromyogram of a 22-year-old male subject who feels oppressive at the right interscapular region. Electrodes were applied to the parts to be tested of the human body diagram on the left in Fig. 5 to measure the dorsal muscular strength and at the same time to record the electromyogram induced from the muscle to be tested. The dorsal muscular strength was recorded on Channel 2, the myoelectric from right and left interscapular regions were recorded on Channels 3 and 4, the myoelectric from right and left lumbodorsal muscles on Channels 5 and 6, all these being, at the same time, supplied to a data recorder.

Studying Fig. 5 it can be seen that the maximum dorsal muscular strength is 138 Kg. This can be understood also from the muscular strength scale. An actual measurement of the maximum height of the curve shows 27.2 mm. Therefore, it is 138 Kg also considering the above calibration voltage. Next, in order to calculate the maximum values of the electromyogrammatic amplitudes the space between the horizontal lines drawn above and below the amplitudes were measured. They were 2.18, 5.24, 1.68 and 1.87 mV for Channels 3, 4, 5 and 6, respectively, in view of the above calibration voltage. These maximum values are all indicated on the panels of Figs. 6 to 8. That is, on the left display window of the panel there is digitally indicated the maximum dorsal muscular strength of 138 Kg, and 2.18, 5.24, 1.68, and 1.87 mV are digitally displayed on the upper display windows of Channels 3, 4, 5 and 6, respectively.

The ratios between the integrated values of the electromyogrammatic and muscular strength signals corrected by the weight/muscular coefficient are displayed on the lower display windows of the panels of Figs. 6 to 8. These labelled AMS ratios correspond to the measurement records of Fig. 5 with the correction value P set for weights (W) of 50 Kg, 25 Kg and 100 Kg, for Figs. 6, 7 and 8, respectively.

Fig. 6 is the case of the weight W=50 Kg. The correction value was set at 50.0. The lower display windows of the panel displayed AMS ratios of 0.71, 1.55, 0.64 and 0.62 mV on Channels 3, 4, 5 and 6, respectively.

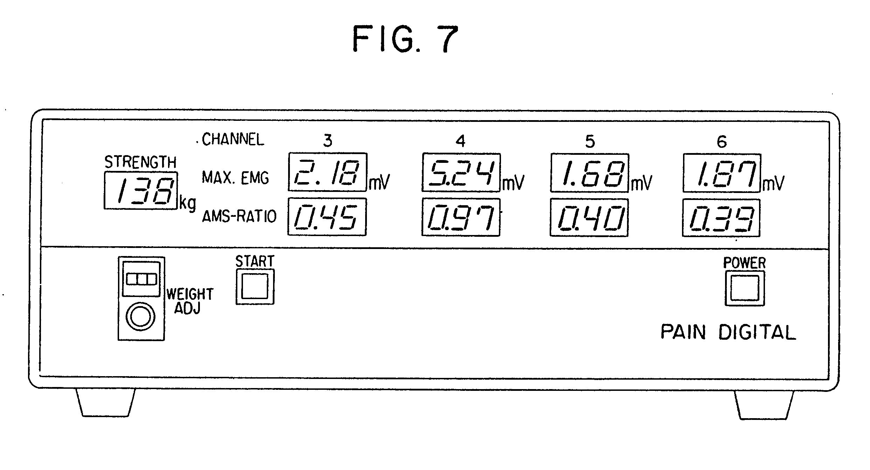

Fig. 7 is the case of the weight W=25 Kg. The correction value was set at 31.5. The lower display windows of the panel displayed AMS ratios of 0.45, 0.97, 0.40 and 0.39 mV on Channels 3, 4, 5 and 6, respectively.

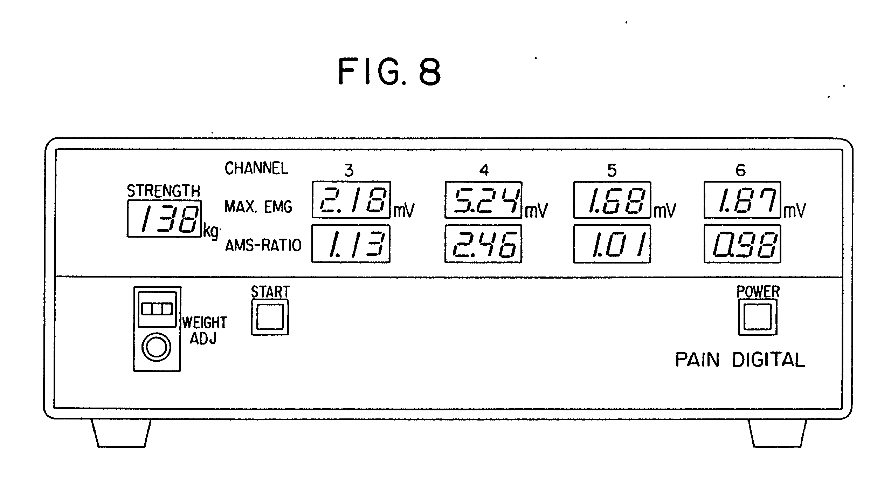

Fig. 8 is the case of the weight W=100 Kg. The correction value was set at 79.4. The lower display windows of the panel displayed 1.13, 2.46, 1.01 and 0.98 mV on Channels 3, 4, 5 and 6, respectively.

The following table is a comparison between the variation in the above displayed values and the increase in the weight.

| Weight Kg | Correction value | Maximum muscular strength Kg | Channel | | | |
|---|---|---|---|---|---|---|
| | | | 3 | 4 | 5 | 6 |
| 25 | 31.5 | 138 | 0.45 | 0.97 | 0.40 | 0.39 |
| 50 | 50.0 | 138 | 0.71 | 1.55 | 0.64 | 0.62 |
| 100 | 79.4 | 138 | 1.13 | 2.46 | 1.01 | 0.98 |

While of the maximum amplitudes at the upper parts of the display panels of Figs. 6 to 8 the discharge amount (maximum electromyogrammatic signal) of 1.87 mV on channel 6 is higher than the figure of 1.68 mV on channel 5, in the lower windows in Fig. 8 channel 5 indicates an AMS ratio of 1.01 mV and channel 16 indicates an AMS ratio of 0.98 mV. Thus, the indication of channel 5 is higher than that of channel 6. This is because the frequency of the electromyogrammatic amplitude was 70 Hz for channel 5 in contrast to 50 Hz for channel 6. For this reason channel 5 displayed a higher value in the integration ratio.

As described above, the inventor has done research and development for a long time on the muscular strength electromyogram in order to measure the fatigue and pain of muscles. As shown in Fig. 5, the fatigue and pain of muscles are clearly numerically expressed. This numerical value is one supporting the appeal of the subject that he feels oppressive at the right interscapular region.

The apparatus of the present invention measures the fatigue and pain muscles and expresses them numerically which no one could have done before. The results can be displayed exactly and in very short time as described above. The operation of the apparatus of the present invention not only permits the results of the processing to be displayed exactly and in a very short time as described above, but also enables the ratio between the integrated values of the electromyogrammatic and muscular strength signals to be digitally displayed after being corrected by a weight/muscular strength coefficient so that the displayed values can be compared under the same condition even if there is imbalance in the displayed values due to the difference in the weight.

5

# 0 074 407

### Claims

1. An apparatus for measuring the extent of muscle fatigue or pain using measurements of muscular strength and myoelectric discharge, characterized in that said apparatus comprises means (4, 5, 15) for transforming the muscular strength of a muscle being examined into an electrical muscular strength signal, means (1, 1', 2, 3, 10—14) for detecting a myoelectric discharge from said muscle to generate an electromyogrammatic signal, first integrator means (19, 20) for integrating said electromyogrammatic signal, second integrator means (19', 20') for integrating said muscular strength signal, and ratio determining means (21) for determining the ratio between the integrated electromyogrammatic signal and the integrated muscular strength signal.

2. An apparatus as claimed in claim 1, further comprising means (18) for adjusting said ratio by a weight-muscular strength coefficient.

3. An apparatus as claimed in claim 2, wherein said means for adjusting comprises means (18) for multiplying said muscular strength signal by $W_0/P$ before the integration thereof by said second integrator means (19', 20'); where P is represented by the formula

$$P = W_0 \times (W/W_0)^{2/3}$$

wherein $W_0$ is a reference weight and W is the weight of the subject being examined.

4. An apparatus as claimed in any one of claims 1 to 3, wherein said first integrator means comprises a first frequency modulator (19) and a first counter (20) arranged to provide a first count value representative of the integrated electromyogrammatic signal, and wherein said second integrator means comprises a second frequency modulator (19') and a second counter (20') arranged to provide a second count value representative of the integrated muscular strength signal.

5. An apparatus as claimed in claim 4, wherein said ratio determining means includes means (21) for dividing said first count value by said second count value to derive said ratio.

6. An apparatus as claimed in any one of the preceding claims, further comprising display means (22) for displaying said ratio.

7. An apparatus as claimed in claim 5 wherein said display means (22) is arranged to display said ratio digitally.

8. An apparatus as claimed in any one of the preceding claims, further comprising means (16) for detecting a maximum amplitude of said electromyogrammatic signal and representing the detected maximum amplitude in a digital value, and means (17) for displaying said digital value by decimal numbers.

9. An apparatus as claimed in any one of the preceding claims, further comprising means (16') for detecting a maximum amplitude of said muscular strength signal and representing the detected maximum amplitude in a digital value, and means (17') for displaying said digital value for said maximum amplitude of said muscular strength signal by decimal numbers.

10. An apparatus as claimed in any one of the preceding claims, wherein said means for detecting a myoelectric discharge comprises (i) electrodes (1, 1') adapted to be placed on said muscle to generate a raw electromyogrammatic (EMG) signal (I) having alternating amplitudes and (ii) waveform processing means comprising filter means (10, 11) for filtering out components of said raw EMG signal having a frequency greater than a first value and less than a second value to produce a filtered EMG signal (III), rectifier means (12) for rectifying said filtered EMG signal to produce a rectified EMG signal (IV), and conversion means (13, 14) for converting said rectified EMG signal into an envelope signal (VI) and for applying a signal representative of the said envelope signal to said first integrator means (19, 20).

11. An apparatus as claimed in claim 10, further comprising delay means (15) to delay said muscular strength signal for a time equal to the duration of said waveform processing of said electromyogrammatic signal by said waveform processing means (10, 11, 12, 13, 14).

12. An apparatus as claimed in either one of claims 10 or 11, wherein said filter means (10, 11) serve to remove from said electromyogrammatic signal components of frequency 10 Hz or less and of frequency 150 Hz or greater.

### Patentansprüche

1. Gerät zur Messung des Ausmaßes von Muskelermüdung oder Muskelschmerz unter Verwendung von Messungen der Muskelstärke oder Muskelfestigkeit und myoelektrischer Abgabe oder Entladung, dadurch gekennzeichnet, daß das Gerät Mittel (4, 5, 15) zum Umwandeln der Muskelstärke oder Muskelfestigkeit eines geprüften Muskels zu einem elektrischen Muskelstärkensignal, Mittel (1, 1', 2, 3, 10—14) zum Feststellen einer myoelektrischen Abgabe oder Entladung von dem Muskel, um ein elektromyogrammatisches Signal zu erzeugen, eine erste Integriereinrichtung (19, 20), um das elektromyogrammatische Signal zu integrieren, eine zweite Integriereinrichtung (19', 20'), um das Muskelstärkensignal zu integrieren, und eine Verhältnisbestimmeinrichtung (21) aufweist, um das Verhältnis zwischen dem integrierten elektromyogrammatischen Signal und dem integrierten Muskelstärkensignal zu bestimmen.

6

2. Gerät nach Anspruch 1, weiterhin umfassend eine Einrichtung (18) zum Einstellen des Verhältnisses durch einen Gewicht-Muskelstärkenkoeffizienten.

3. Gerät nach Anspruch 2, dadurch gekennzeichnet, daß die Einstelleinrichtung Mittel (18) aufweist zum Multiplizieren des Muskelstärkensignals mit $W_0/P$ vor dem Integrieren dieses Signales durch die zweite Integriereinrichtung (19', 20'), wobei P wiedergegeben ist durch die Formel

$$P=W_0\times(W/W_0)^{2/3},$$

worin $W_0$ ein Bezugsgewicht, und W das Gewicht des geprüften Subjektes ist.

4. Gerät nach irgendeinem der Ansprüche 1 bis 3, wobei die erste Integriereinrichtung einen ersten Frequenzmodulator (19) und einen ersten Zähler (20) aufweist, um einen ersten Zählwert zu schaffen, der für das integrierte elektromyogrammatische Signal repräsentativ ist, und wobei die zweite Integriereinrichtung einen zweiten Frequenzmodulator (19') und einen zweiten Zähler (20') aufweist, um einen zweiten Zählwert zu schaffen, der für das integrierte Muskelstärkensignal repräsentativ ist.

5. Gerät nach Anspruch 4, wobei die Verhältnisbestimmeinrichtung Mittel (21) aufweist zum Dividieren des ersten Zählsignals durch das zweite Zählsignal, um das Verhältnis abzuleiten.

6. Gerät nach irgendeinem der vorhergehenden Ansprüche, weiterhin umfassend eine Anzeigeeinrichtung (22) zum Anzeigen dieses Verhältnisses.

7. Gerät nach Anspruch 5, wobei die Anzeigeeinrichtung (22) so ausgeführt ist, daß sie das Verhältnis digital anzeigt.

8. Gerät nach irgendeinem der vorhergehenden Ansprüche, weiter umfassend eine Einrichtung (16) zum Feststellen einer maximalen Amplitude des elektromyogrammatischen Signales und zum Darstellen der festgestellten maximalen Amplitude mit einem digitalen Wert, und eine Einrichtung (17) zum Anzeigen des digitalen Wertes durch Dezimalzahlen.

9. Gerät nach irgendeinem der vorhergehenden Ansprüche, weiter umfassend eine Einrichtung (16') zum Feststellen einer maximalen Amplitude des Muskelstärkensignals und Darstellen der festgestellten maximalen Amplitude mit einem digitalen Wert, und eine Einrichtung (17') zum Anzeigen des digitalen Wertes für die maximale Amplitude des Muskelstärkensignales mit Dezimalzahlen.

10. Gerät nach irgendeinem der vorhergehenden Ansprüche, wobei die Einrichtung zum Feststellen einer myoelektrischen Abgabe oder Entladung (i) Elektroden (1, 1'), die an dem Muskel angeordnet werden können, um ein grobes elektromyogrammatisches (EMG) Signal (I) zu erzeugen, welches wechselnde Amplituden hat, und (ii) eine Wellenformverarbeitungseinrichtung aufweist, die eine Filtereinrichtung (10, 11) zum Ausfiltern von Komponenten des groben EMG-Signals, die eine Frequenz größer als ein erster Wert und kleiner als ein zweiter Wert haben, um ein gefiltertes EMG-Signal (III) zu erzeugen, eine Gleichrichteinrichtung (12) zum Gleichrichten des gefilterten EMG-Signals, um ein gleichgerichtetes EMG-Signal (IV) zu erzeugen, und eine Umwandlungseinrichtung (13, 14) aufweist, um das gleichgerichtete EMG-Signal zu einem Hüllsignal (VI) umzuwandeln und um ein Signal, welches für das Hüllsignal repräsentativ ist, an die erste Integriereinrichtung (19, 20) anzulegen.

11. Gerät nach Anspruch 10, weiter umfassend eine Verzögerungseinrichtung (15), um das Muskelstärkensignal für eine Zeit zu verzögern, die gleich der Dauer der Wellenformverarbeitung des elektromyogrammatischen Signals durch die Wellenformverarbeitungseinrichtung (10, 11, 12, 13, 14) ist.

12. Gerät nach Anspruch 10 oder 11, wobei die Filtereinrichtung (10, 11) dazu dient, aus dem elektromyogrammatischen Signal Komponenten einer Frequenz von 10 Hz oder weniger und einer Frequenz von 150 Hz oder höher zu entfernen.

**Revendications**

1. Appareil pour mesurer le degré de fatigue ou de douleur d'un muscle en utilisant des mesures d'une force musculaire et d'une décharge myoélectrique, caractérisé en ce que ledit appareil comprend des moyens (4, 5, 15) destinés à transformer la force musculaire d'un muscle examiné en un signal électrique de force musculaire, des moyens (1, 1', 2, 3, 10—14) destinés à détecter une décharge myoélectrique dudit muscle pour générer un signal électromyographique, des premiers moyens intégrateurs (19, 20) destinés à intégrer ledit signal électromyographique, des seconds moyens intégrateurs (19', 20') destinés à intégrer ledit signal de force musculaire, et des moyens (21) de détermination de rapport destinés à déterminer le rapport du signal électromyographique intégré et du signal intégré de force musculaire.

2. Appareil selon la revendication 1, comprenant en outre des moyens (18) destinés à ajuster ledit rapport par un coefficient poids-force musculaire.

3. Appareil selon la revendication 2, dans lequel lesdits moyens d'ajustement comprennent des moyens (18) destinés à multiplier ledit signal de force musculaire par $W_0/P$ avant son intégration par lesdits seconds moyens intégrateurs (19', 20'); où P est représenté par la formule

$$P=W_0\times(W/W_0)^{2/3}$$

dans laquelle $W_0$ est un poids de référence et W est le poids du sujet examiné.

4. Appareil selon l'une quelconque des revendications 1 à 3, dans lequel lesdits premiers moyens

# 0 074 407

intégrateurs comprennent un premier modulateur de fréquence (19) et un premier compteur (20) agencé pour produire une première valeur de compte représentative du signal électromyographique intégré, et dans lequel lesdits seconds moyens intégrateurs comprennent un second modulateur de fréquence (19') et un second compteur (20') agencés pour produire une seconde valeur de compte représentative du signal de force musculaire intégré.

5. Appareil selon la revendication 4, dans lequel lesdits moyens de détermination de rapport comprennent des moyens (21) destinés à diviser la première valeur de compte par ladite seconde valeur de compte afin d'établir ledit rapport.

6. Appareil selon l'une quelconque des revendications précédentes, comprenant en outre des moyens d'affichage (22) destinés à afficher ledit rapport.

7. Appareil selon la revendication 5, dans lequel lesdits moyens d'affichage (22) sont agencés pour afficher numériquement ledit rapport.

8. Appareil selon l'une quelconque des revendications précédentes, comprenant en outre des moyens (16) destinés à détecter une amplitude maximale dudit signal électromyographique et à représenter par une valeur numérique l'amplitude maximale détectée, et des moyens (17) destinés à afficher ladite valeur numérique en chiffres décimaux.

9. Appareil selon l'une quelconque des revendications précédentes, comprenant en outre des moyens (16') destinés à détecter une amplitude maximale dudit signal de force musculaire et à représenter l'amplitude maximale détectée par une valeur numérique, et des moyens (17') destinés à afficher ladite valeur numérique de ladite amplitude maximale dudit signal de force musculaire en chiffres décimaux.

10. Appareil selon l'une quelconque des revendications précédentes, dans lequel lesdits moyens destinés à détecter une décharge myoélectrique comprennent (i) des électrodes (1, 1') conçues pour être placées sur ledit muscle afin de générer un signal électromyographique brut (EMG) (I) possédant des amplitudes alternantes et (ii) des moyens de traitement de formes d'ondes comprenant des moyens de filtrage (10, 11) destinés à éliminer par filtrage des composantes dudit signal EMG brut ayant une fréquence supérieure à une première valeur et inférieure à une seconde valeur afin de produire un signal EMG filtré (III), des moyens redresseurs (12) destinés à redresser ledit signal EMG filtré pour produire un signal EMG redressé (IV), et des moyens de conversion (13, 14) destinés à convertir ledit signal EMG redressé en un signal d'enveloppe (VI) et à appliquer un signal représentatif dudit signal d'enveloppe auxdits premiers moyens intégrateurs (19, 20).

11. Appareil selon la revendication 10, comprenant en outre des moyens de retard (15) destinés à retarder ledit signal de force musculaire pendant un temps égal à la durée dudit traitement de la forme d'onde dudit signal électromyographique par lesdits moyens de traitement de formes d'ondes (10, 11, 12, 13, 14).

12. Appareil selon l'une des revendications 10 et 11, dans lequel lesdits moyens de filtrage (10, 11) servant à éliminer dudit signal électromyographique des composantes d'une fréquence de 10 Hz ou moins et d'une fréquence de 150 Hz ou plus.

8

FIG. 1

# FIG. 2

# FIG. 3

0 074 407

# FIG. 4

15

A

B

19'

R

R

+

−

100

D

50

Ad

0

4

FIG. 5

FIG. 6

# FIG. 7

PAIN DIGITAL

CHANNEL 3 4 5 6

STRENGTH
*138* kg

MAX. EMG   *2.18* mV   *5.24* mV   *1.68* mV   *1.87* mV

AMS-RATIO   *0.45*   *0.97*   *0.40*   *0.39*

WEIGHT ADJ   START   POWER

0 074 407

# FIG. 8

CHANNEL

STRENGTH

*138* kg

|        | 3 | 4 | 5 | 6 |
|--------|---|---|---|---|
| MAX. EMG | *2.18* mV | *5.24* mV | *1.68* mV | *1.87* mV |
| AMS-RATIO | *1.13* | *2.46* | *1.01* | *0.98* |

START

WEIGHT
ADJ

POWER

PAIN  DIGITAL

0 074 407

## FIG. 9